# EUROPEAN PATENT SPECIFICATION

(11) **EP 0 476 953 B1**
(45) Date of publication and mention of the grant of the patent: **17.03.1999**
(21) Application number: 91308438.0
(22) Date of filing: 16.09.1991
(51) Int. Cl.: A61K 48/00

(54) **Targeted destruction of neoplastic cells by retroviral vector-producing packaging cells**
Gezielte Zerstörung neoplastischer Zellen mit Hilfe von Retrovirusvektor-produzierenden Packungszelllinien
Destruction ciblée de cellules néoplastiques à l'aide de cellules de packaging produisant des vecteurs rétroviraux

(30) Priority: 14.09.1990 US 582055; 16.08.1991 US 746655
(43) Date of publication of application: 25.03.1992
(73) Proprietor: THE GENERAL HOSPITAL CORPORATION, Boston, Massachusetts 02114 (US)
(72) Inventor: Martuza, Robert L., Lexington, Massachusetts 01273 (US); Breakfield, Xandra O., Newton, Massachusetts 02159 (US)
(74) Representative: Sheard, Andrew Gregory

(56) References cited:
- CHEMICAL ABSTRACTS, vol. 110, no. 7, February 13, 1989, Columbus, Ohio, USA I. J. FIDLER et al. "Recogni- tion and destruction of neo- plastic cells by activated macrophages: discrimination of altered self" page 496, abstract-no. 55 431m
- J. of the National Cancer Institute 84(4), 297-300 (1990)

## Description

This invention relates to treatment of neoplastic cells utilizing retroviral vector-producing packaging cells.

Neoplasia is a process by which the normal controlling mechanisms that regulate cell growth and differentiation are impaired resulting in progressive growth. During neoplasia, there is a characteristic failure to control cell turnover and growth. This lack of control causes a tumor to grow progressively, enlarging and occupying spaces in vital areas of the body. If the tumor invades surrounding tissue and is transported to distant sites the tendency of this tumor will be to result in death of the individual.

One-third of all individuals in the United States will develop cancer (American Cancer Society Yearly Outlook for 1990). The five year survival rate for these patients has risen to nearly 50% as a result of progress and early diagnosis and therapy of the disease (American Cancer Society Yearly Outlook for 1990). However, cancer remains second only to cardiac disease as a cause of death in this country (American Cancer Society Yearly Outlook for 1990). Nearly 20% of all Americans who die this year will die of cancer (American Cancer Society Yearly Outlook for 1990). Half of these deaths will be due to the three most common types of cancer: lung, breast, and colon.

Recently there has been a rapid expansion of cancer treatments. Even though new treatments are being developed, the need still exists for improved methods for the treatment of most types of cancers.

The preferential killing of cancer cells without deleterious effect on normal cells is the desired goal in cancer therapy. In the past this has been accomplished using a variety of procedures. These procedures include the administration of chemicals, chemotherapy, radiation, radiotherapy, and surgery.

Radiotherapy is a regional form of treatment used for the control of localized cancers (See Devita, V.T., in Harrisson's *Principles of Internal Medicine*, ed. Braunwald *et al*., 1987, McGraw-Hill Inc., New York, p. 431-446). Radiotherapy relies on the fact that some malignant diseases are more susceptible to damage by radiation. This difference in susceptibility depends on normal cells having a higher capacity for intercellular repair than neoplastic cells and the ability of normal organs to continue to function well if they are only segmentally damaged. If surrounding tissue can tolerate twice the radiation dose of a given tumor, then the tumor is radiosensitive. On the other hand, some tumors cannot be treated with radiotherapy. Cancer which extensively involves both lungs cannot be treated effectively with radiation therapy because of the greater radio-sensitivity of the surrounding lung tissue (See Devita, V.T., in Harrison's *Principles of Internal Medicine*, ed. Braunwald *et al*., 1987, McGraw-Hill Inc., New York, p. 431-446).

Surgery is still considered the primary treatment for most early cancers (See Devita, V.T., in Harrison's *Principles of Internal Medicine*, ed. Braunwald *et al*., 1987, McGraw-Hill Inc., New York, p. 431-116). However, most tumors are operable but not fully resectable. Some tumors that appear resectable have micrometastatic disease outside the tumor field. This leads to a recurrence of the cancer close to the initial site of occurrence. Any cancer showing a level of metatastis effectively cannot be cured through surgery.

Other types of localized therapy (nonsystemic) have been explored. These include local hypothermia (Saloman *et al., J. Neuro-Oncol. 1*:225-236 (1983)), photodynamic therapy (Cheng *et al., Surg. Neurol. 25*:423-435 (1986)), and interstitial radiation (Gutin *et al., J. Neurosurgery 67*:864-873 (1987)). To date these therapies have been met with limited success.

Radiotherapy and surgery offers ways of reducing the tumor mass in specific regions of the body that are accessible through surgical techniques or high doses of radiotherapy. Neither is applicable to the destruction of widely disseminated or circulating tumor cells characteristically present in most patients with cancer. This is the stimulus of the development of systemic treatments of cancer such as chemotherapy.

The use of chemicals, even though widespread in use, has proved limitedly effective in treating most cancer types. One drawback to the use of cytotoxic agents for the treatment of cancer are their severe side effects. These include nausea, vomiting, CNS depression, localized pain, bone marrow depression, bleeding, renal damage, hypo and hyperglycemia and hypersensitivity reactions. Another drawback is that they are only effective against rapidly dividing cells.

A more modem approach to chemotherapy is to direct the toxic agents to the cancer cells themselves. This has been accomplished experimentally by linking the chemotherapeutic agent to either antibodies or toxic molecules that have a higher affinity for the tumor cells than normal cells. These directed toxic bullets are still in an early clinical phase of development and are not commercially available.

Certain types of cancer, for example gliomas, which are the most common primary tumor arising in the human brain, defy the current modalities of treatment. Despite surgery, chemotherapy, and radiotherapy, glyomablastoma, the most common of the gliomas is almost universally fatal (Schoenberg, B.S., "The epidemiology of nervous system tumors." in *Oncology of the Nervous System,* M.D. Walker, ed., Boston, MA, Martinus Nijhoff (1983); Levin *et al*., "Neoplasms of the Central Nervous System," Chapter 46, pp. 1557-1611, in *Cancer: Principles and Practice of Oncology,* vol 2, 3^{rd} ed., De Vita *et al*., eds., Philadelphia, Lippincott Press (1989)). Therefore a need exists for the development of a technique that will selectively destroy glioma while sparing normal brain cells. In general, such treatment could potentially be used universally for the selective destruction of all types of neoplastic cells.

Compositions are provided for selectively killing neoplastic cells. In particular, retroviral vector-producing packaging cells are utilized to target expression of a gene or gene product in neoplastic cells. Genes are selected whose gene products are capable of targeting cells for selective killing of tumour cells. The present invention finds particular use in the treatment of tumours of the nervous system.

According to a first aspect of the present invention there is provided the use of retroviral vector-producing packaging cells in the preparation of an anti-neoplastic cell agent or of a sensitizing agent against neoplastic cells already existing in a patient, wherein the retroviral vector produced by said packaging cells can infect neoplastic cells, and wherein said retroviral vector contains a gene whose gene product is capable of:
1) killing the neoplastic cells, or
2) sensitizing the neoplastic cells so that they can be killed by additional chemical treatment or radiation.

The invention also contemplates retroviral vector-producing packaging cells for use in medicine, wherein the retroviral vector produced by said packaging cells can infect neoplastic cells already existing in a patient, and wherein said retroviral vector contains a gene whose gene product is capable of:
1) killing the neoplastic cells, or
2) sensitizing the neoplastic cells so that they can be killed by additional chemical treatment or radiation.

Additionally, the invention relates to a pharmaceutical composition comprising:
a) retroviral vector-producing packaging cells, wherein the retroviral vector produced by said packaging cells can infect neoplastic cells already existing in a patient, and wherein said retroviral vector contains a gene whose gene product is capable of:
   1) killing the neoplastic cells, or
   2) sensitizing the neoplastic cells so that they can be killed by additional chemical treatment or radiation, and
b) a pharmaceutically acceptable (e.g. sterile) carrier.

This composition may be an injectable composition, for example a sterile solution.

The invention additionally contemplates processes for the preparation of such pharmaceutical compositions, the process comprising admixing the retroviral vector producing packaging cells and the pharmaceutically acceptable carrier.

The invention may additionally encompass a product comprising:
a) retroviral vector-producing packaging cells, wherein the retroviral vector produced by said packaging cells can infect neoplastic cells already existing in a patient, and wherein said retroviral vector contains a gene whose gene product is capable of:
   1) killing the neoplastic cells, or
   2) sensitizing the neoplastic cells so that they can be killed by additional chemical treatment or radiation, and
b) a chemical for use in said chemical treatment,
as a combined preparation for simultaneous, sequential or separate use in tumour therapy.

The retrovirus vector produced by packaging cells used in the present application will usually only integrate into the genome of dividing cells. The vector may thus selectively target dividing (e.g. neoplastic) cells and comprises a gene which produces a gene product in the cell. The gene or gene product may thus be adapted to directly or indirectly kill neoplastic cells or sensitise them so that they can be killed by additional chemical treatment or by radiation.

The chemical treatment may include treatment with antibodies, an agent which recognises the gene product and may thus selectively kill the neoplastic cell or a nucleoside analogue such as acyclovir, gancyclovir or FIAU.

The invention contemplates a considerable variety of ways in which a neoplastic cell can be killed or sensitised so that it can be killed by additional chemical treatment or by radiation. It will now be briefly discussed.

As far as the gene product is concerned, this may have any or all of the following properties:
(1) it may sensitise the cell for further treatment (such as radiation or chemical treatment);
(2) it may kill the cell, such as by being an antisense nucleic acid (for example encoding an essential protein) or by being a toxic protein.

As far as the gene itself is concerned, this may have any or all of the following properties:
(1) it may be toxic to the cell;
(2) it may render the cell more antigenic (xenogenisation of the cell) for example by causing expression of non-human and/or unique surface antigens, which may allow further chemical treatment, such as using antibodies and preferably labelled antibodies in order to try to kill the cell; and
(3) it may provide a cell killing mechanism, for example the gene may comprise the herpes simplex virus I thymidine kinase gene.

The retrovirus may be delivered by grafting a packaging cell line. In preferred embodiments the neoplastic cell may be later contacted with radiation.

Preferably a helper virus may also be provided, for example in a pharmaceutical composition and therefore will usually accompany the retroviral vector-producing packaging cells. The helper virus will therefore serve the purpose of preferably co-infecting thereby augmenting gene delivery. The helper virus is suitably a ecotropic (such as wild type) retrovirus. In some embodiments the retroviral vector-producing packaging cells will also be transfected with the helper virus.

The present invention is thus drawn to the selective killing of neoplastic cells. Retroviral vector-producing packaging cells capable of producing vectors carrying a gene whose gene product is capable of killing or targeting neoplastic cells for selective cell death are utilised.

By neoplastic cells is intended dividing cells, usually rapidly dividing cells. For purposes of the invention, neoplastic cells include cells of tumors, neoplasms, carcinomas, sarcomas, leukemias, lymphomas, and the like. Of particular interest are central nervous system tumors. These include astrocytomas, oligodendrogliomas, meningiomas, neurofibromas, ependymomas, Schwannomas, neurofibrosarcomas, glioblastomas, etc. The neoplastic cells of particular concern to the invention are those cells of brain tumors. Adult brain tumors are unique in that they constitute masses of dividing cells within a background of essentially non-dividing cells. Therefore, the present invention utilizes these metabolic differences to exploit the development of a targeted approach to selective killing of neoplastic cells. The invention can be utilized to selectively kill both benign and malignant neoplastic cells.

The retroviral vectors that can be produced by the retroviral vector-producing packaging cells of the invention can integrate only into the genome of dividing cells. Thus, the vectors provide a useful vehicle for selective targeting of dividing cells. Retroviral vectors offer further advantages as there are no limitations in host range and these vectors have already been used successfully to infect many different cell types. For example, see Cepko, C., "Lineage analysis and immortalization of neural cells via retrovirus vectors," in *Neuromethods*, Vol. 16, pp. 177-218, Clifton, NJ, The Humana Press, Inc. (1989); Gilboa, E., *BioEssays 5(6)*:252-257 (1987); Friedmann T., *Science 244*:1275-1281(1989).

In general, retroviral vectors are well known in the art. See Breakfield *et al*., *Molec*. *Neuro. Biol*. *1*:339 (1987); and, Shih *et al*., In: *Vaccines 85*, Cold Spring Harbor Press, Cold Spring Harbor, New York (1985) pages 177-180. Further, US Patent No. 5501979 is drawn to herpes simplex virus expression vectors.

They provide further information on the construction and use of retrovirus vectors.

As indicated above, generally, the retrovirus vectors that can be produced by the retroviral vector-producing packaging cells of the present invention are replication-defective. They can be packaged into infectious retroviral particles by transfected cell lines which contain retroviral sequences coding for the proteins necessary for the packaging of retroviral RNA but which cannot package their own RNA See, Mann *et al*, *Cell 33*:153-159 (1983); Danos and Mulligan, *Proc*. *Natl*. *Acad. Sci. USA 85*:6460-6464 (1988). Since retrovirus and vectors derived from them integrate into the host cell genome, their sequences are transmitted to all daughter cells. This feature of retroviruses has been successfully used for example, to trace cell lineages in the nervous system (Price *et al*., *Proc. Natl. Acad*. *Sci. USA 84*:156-160 (1987); Luskin *et al*., *Neuron 1*:635-647 (1988); Walsh and Cepko, *Science 241*:1342-1345(1988)).

Genes for transfer into the neoplastic cells by retroviral vectors produced by packaging cells are selected from those which target the host cell usually by the expression of a gene product in the host neoplastic cells. "Gene product" broadly refers to proteins encoded by the particular gene. However, for purposes of the invention, gene product also includes transcription products of the gene, particularly for use as anti-sense RNA. The host cells targeted by the present invention are those cells into which the virus produced by packaging cells infects and expresses the desired gene product. The host cells thus constitute neoplastic cells infected by the retroviral vectors produced by packaging cells.

Genes are selected whose gene products kill host cells and/or whose products sensitise the host cell for cell death. Cell death can be accomplished by contacting the host cells, comprising the gene product, with a subsequent treatment, either radiation or chemical treatment. Alternatively, the gene products themselves may serve to kill the host cells.

Gene products themselves capable of selective cell killing may comprise anti-sense nucleic acid for essential cell proteins, such as replication proteins, which serve to render the host cells incapable of further cell growth and division. Anti-sense regulation has been described by Rosenberg *et al., Nature 313*:703-706 (1985); Preiss *et al., Nature 313*:27-32 (1985); Melton, *Proc. Natl. Acad. Sci. USA 82*:144-148 (1985); **Izant and Weintraub**, *Science 229*:345-352 (1985); Kim and Wald, *Cell 42*:129-138 (1985); Pretka *et al., Proc. Natl. Acad. Sci. USA 81*:7525-7528 (1984); Coleman *et al., Cell 37*:683-691 (1984); and McGarry and Lindquist, *Proc. Natl. Acad. Sci. USA 83*:399-403 (1986).

Other genes which find use for slowing cell growth include tumor suppressor genes, genes which encode transcription factors which suppress cell growth, toxic proteins that are released by cells, and the like. For example, see Heinbrook *et al., Proc. Natl. Acad. Sci. USA 87*:4697 (1990), which describes a fusion protein with toxin coupled to the EGF ligand. Toxin genes have also been described, for example, Barker *et al., Gene 86*:285-290 (1990); Ito *et al., Microb. Pathog. 8*:47-60 (1990); Gannon *et al., J. Gen. Microbiol 136*:1125-1136 (1990). Genes can also be inserted which alter cell growth characteristics or modulate cell growth, for example, a tumor suppressor gene such as the Rb gene in retinoblastoma (Huang *et al, Science 242*:1563-1566 (1988)) or the p53 gene in colon cancer (Baker *et al., Science 249*:912-915 (1980)). Other suppressor or modulating genes may also be utilized.

Genes whose products serve to render the host cells more antigenic also find use in the invention. This antigenic effect may be accomplished by introducing new antigens on the surface of the host cells, thus augmenting the immune system in recognizing the tumor as a foreign body. The introduction of new antigens to the surface of the host cells is referred to as xenogenization of the cells (Austin *et al., Ad. in Cancer Res. 30*:301-345 (1979); Kobayashi *et al., Ad. in Cancer Res. 30*:279-299 (1979)). Any nonhuman surface antigen can be utilized including those described in Araki *et al., Gene 89*: 195 -202 ( 1990); Takle *et al., Mol. Biochem.* *Parasitol*. 37:57-64 (1989); Raney *et al*., *J. Virol. 63*:3919-3925 (1989); Tondravi, M.M., *Curr. Genet. 14*:617-626 (1988); and Miyandhara *et al*., *Proc. Natl. Acad*. *Sci*. *USA 80*:1-5(1983).

A gene or coding sequence may be selected whose products offers a conditional killing mechanism for dividing cells. In this manner, the expression of a particular protein followed by the subsequent treatment is effective in killing the neoplastic cells. The subsequent treatment comprises chemical treatment or radiation. Agents for chemical treatments comprise the use of enzymes or other compounds which react with the gene product to kill the host cell.

For example, the herpes simplex virus type I (HSV-1) thymine kinase (TK) gene offers such a conditional killing mechanism for dividing cells. The selective advantage of using HSV-1-TK derived from the fact that the enzyme has a higher affinity for certain nucleoside analogues, such as acyclovir, gancyclovir and FIAU, than mammalian TK (McLaren *et al*., In: *Herpes Virus and Virus Chemotherapy*, R. Kono, ed., pp.57-61, Amsterdam, Elsevier (1985)). These drugs are converted to nucleotide-like precursors and incorporated into the DNA of replicating cells, thus disrupting the integrity of the genome, and ultimately leading to cell death. Several studies have successfully made use of the conditional toxicity of TK in development studies of transgenic mice (Borrelli *et al*., *Nature 339*:538-541 (1989); Heyman *et al*., *Proc. Natl. Acad. Sci. USA* *86*:2698-2702 (1989)), as a selectable marker against non-homologous recombination events in cultured cells (Capecchi, M.R., *Trends in Genetics 5(3)*:70-76 (1989)), for killing cells harboring wild type herpes viruses (Corey and Spear, *N. Engl. J. Med 314*:686-691 (1986); Corey and Spear, *N. Engl J. Med. 314*:749-756 (1986)), and in selecting for herpes virus mutants lacking TK activity (Coen *et al*., *Science 234*:53-59 (1986)).

The gene product may also encode a chemical or protein which renders the host cells radiosensitive and thus more susceptible to killing by radiation. Thus, upon subsequent subjection to radiation, the host cells are selectively killed. For example, see Snyderman *et al., Arch*. *Otolaryngol. Head Neck Surg. 112*:1147-1150 (1986); and Sealy *et al., Cancer 54*:1535-1540 (1984). Other strategies include selective transfer of cell surface antigenic markers, in conjunction with the development of tumor-specific immunoconjugates to improve targeting of chemotherapeutic agents. See, Reisfeld, R.A., in *Molecular Probes Technology and Medical Applications,* Albertini, A. *et al.,* Raven Press, New York (1989).

It is recognized that the gene of interest may be modified by any methods known in the art. For example, the gene may be placed under the control of heterologous regulatory regions, including the use of viral promoters, neoplastic cell or tumor specific promoters or control elements. In this manner, the gene product is further targeted to specific cell types. Methods for construction of such expression vectors are known in the art.

Generally, methods are known in the art for retroviral infection of the cells of interest. Typically, the virus is injected into the host at or near the site of neoplastic growth. For the most part, the virus is provided in a therapeutically effective amount to infect and kill target cells. Generally, the virus is provided for injection in a concentration in the range of about 10¹ to about 10¹⁰ plaque forming units (PFU), generally about 5x10⁴ to about 1x10⁶ PFU, more generally about 1x10⁵ to about 4x10⁵, although ranges may vary. Alternatively, the packaging cell line may be grafted near or into the tumor to provide a longer-lasting source of virus.

This selective killing of the retrovirus and delivery of the toxic gene can be enhanced by co-infection with a helper virus. That is, the helper virus augments gene delivery. In this manner, the packaging cell lines for making virus particles of the retrovirus vectors can be coinfected with a helper virus. Packaging cells are then injected into the host at or near the site of infection. (See, Cepko, C. (1989), *supra*; Rosenberg *et al*., *Science 242*:1575-1578 (1988); and Mann *et al*., *Cell 33*:153-159 (1983)). Such helper viruses include ecotropic wild-type retroviruses, for example MoMLV (See, Danos *et al*., *Proc. Natl. Acad. Sci. USA 85*:6460-6464 (1988); Cepko, C., In *Neuromethods, Vol. 16*, *Molecular Neurobiological Techniques*, Boulton *et al.* (eds.), Clifton, NJ: Humana (1989); and Mann *et al*., *Cell 33*:153-159 (1983)).

To utilize a helper virus, the packaging line can be subsequently infected with wild-type virus in culture and these cells can be grafted. (See, Rosenberg *et al*., *Science 242*:1575-1578 (1988) and Wolff *et al*., *Proc*. *Natl*. *Acad. Sci*. *USA 86*:9011-9014 (1989)). The packaging cells are infected with the helper in the range of MOI of about 0.1 to about 20.

The sensitivity of the tumor cells to toxic agents is increased utilizing helper viruses. The helper viruses turn cells infected with retrovirus vectors into packaging cell lines. The results show that by co-infection with a helper virus, the retrovirus vectors of the invention are able to target more tumor cells, even those tumor cells away from the tumor mass. Furthermore, the tumor cells die faster and show more sensitivity to toxic agents when a helper virus is utilized.

The invention finds particular use in the treatment of glioblastomas. Glioblastomas are the most common form of malignant brain tumor in man, and are almost always universally fatal. The glioblastoma represents approximately 30% or 50% of all primary brain tumors and, despite surgery, chemotherapy, and radiotherapy, are almost universally fatal. The mean survival is less than a year, and the five-year survival rate is only 3% or 5%. After treatment, reoccurrence of the disease often appears within two centimeters of the original site. Metastases are extremely rare; neurological disfunction and death are due to local growth and cerebral invasion. Therefore, the possible efficacy of local (non-systemic) treatments has been explored. A few of these include studies of local hypothermia, photodynamic therapy, and interstitial radiation. Until the present invention, no therapeutic modality has made a substantial impact on the outcome of patients with malignant gliomas.

The invention will now be described by way of example with reference to the accompanying drawings, in which:

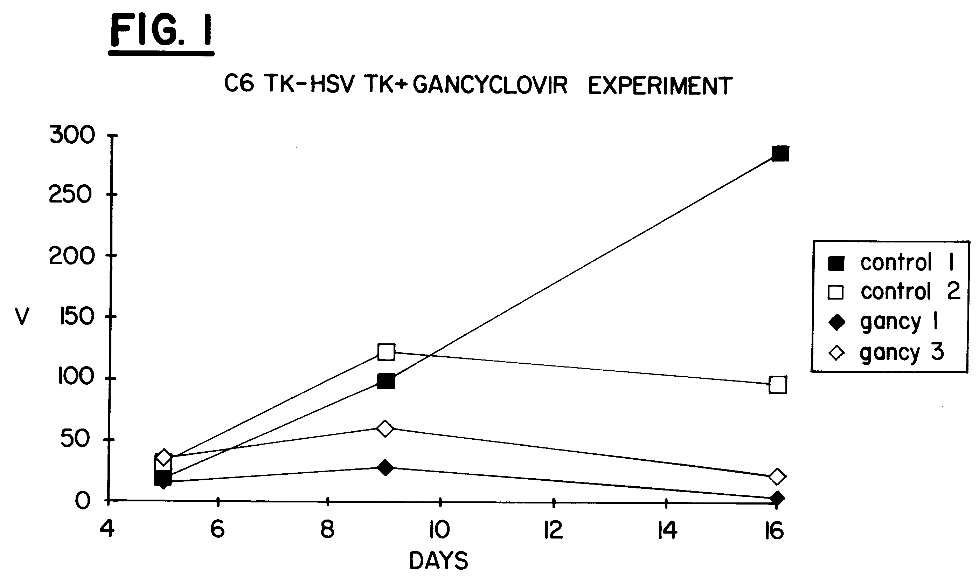

FIGURE 1 is a graph showing the *in vivo* study of gancyclovir; and

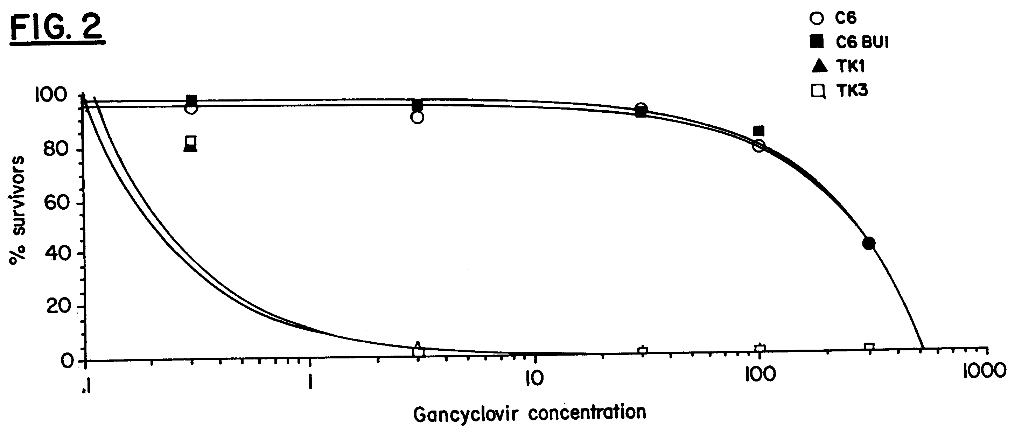

FIGURE 2 is a graph of gancyclovir concentration against percentage survival in a gancyclovir sensitivity assay.

The invention will now be described with reference to the following Examples which are offered by way of illustration and are not to be construed as being limiting on the present invention. (Where examples are not within the scope of the present claims then they are provided for reference purposes.)

### EXAMPLE 1

Primary human brain tumors (malignant gliomas) are not encapsulated, and it is therefore difficult to ensure their complete removal surgically. Many of these tumors are non-metastatic and may, at times, only invade a few centimeters into the surrounding tissue. However, surgery, radiotherapy and chemotherapy have only had a modest impact on the overall morbidity and mortality of affected individuals. Novel, targeted approaches to the treatment of malignant gliomas are worthy of exploration.

Brain tumors are unique in that they constitute masses of dividing cells within a background of essentially non-dividing cells. These metabolic differences can be exploited in the development of targeted approaches to therapy. Retroviral vectors provide a useful vehicle for selective targeting since (1) they can only integrate into the genome of dividing cells; (2) there are no limitations in host range; and (3) these vectors have already been used successfully to infect many different cell types (for review, see Cepko, C., In *Neuromethods, Vol. 16: Molecular Neurobiological Techniques,* Boulton,AA., *et al.,* (eds.), Clifton, N.J., The Humana Press, pp. 177-218 (1989); Gilboa, E., *BioEssays 5*:252-257 (1987); Friedmann, T., *Science 244*:1275-1281 (1989)). The retrovirus vector are replication-defective and can be packaged into infectious retroviram particles by transfected cell lines which contain retroviral sequences coding for the proteins necessary for the packaging of retroviral RNA, but which cannot package their own RNA (e.g., Mann, R., *et al., Cell 33*:153-159 (1983); Danos, O., *et al.,Proc. Natl. Acad. Sci. USA 85*:6460-6464 (1988)). Since retroviruses and vectors derived from them integrate into the host cell genome, their sequences are transmitted to all daughter cells. This feature of retoviruses has been successfully used, for example, to trace cell lineages in the nervous system (Price, J., *et al., Proc Natl. Acad. Sci. USA 84*:156-160 (1987); Luskin, M.B., *et al., Neuron 1*:635-647 (1988); Walsh, C., *et al., Science 241*:1342-1345 (1988)).

The herpes simplex virus type 1 (HSV-1) thymidine kinase (TK) gene offers a conditional killing mechanism for dividing cells. The selective advantage of using HSV-1-TK derives from the fact that this enzyme has a higher affinity for certain nucleoside analogs, such as acyclovir, gancyclovir and FIAU, than mammalian TK (McLaren C., *et al.,* In *Herpes Virus and Virus Chemotherapy*, Kono, R. (ed.), Amsterdam: Elsevier, pp. 57-61 (1985)). These drugs are converted to nucleotide-like precusors and incorported into the DNA of replicating cells, thus disrupting the integrity of the genome, and ultimately leading to cell death. Several studies have sucessfully made use of its conditional toxicity in developmental studies of transgenic mice (Borelli, E., *et al., Proc. Natl. Acad. Sci. USA 85*:7572-7576 (1988); Heyman, R.A., *et al., Proc. Natl. Acad. Sci. USA 86*:2698-2702 (1989)) as a selectable marker against non-homologous recombination events in cultured cells (Capecchi. M.R., *Trends in Genetics 5(3)*:70-76 (1989)), for killing cells harboring wild-type herpes viruses (Corey, L., *et al., N. Engl J. Med. 314*:686-691 (1986); Corey, L., *et al., N. Engl. J. Med. 314*:749-756 (1986)), and in selecting for herpes virus mutants lacking TK activity (Coen, D.M., *et al., Science 234*:53-59 (1986)).

In this study we used rat C6 glioma cells as a model primary brain tumor type. C6 cells rapidly form a non-encapsulated, non-metastatic tumor after injection into the adult rat CNS. Further, derivative cell lines are available, which lack endogenous TK activity (C6-BUI) or bear the *lacZ* gene (C6-BAG), which are useful experimentally. A retroviral vector was generated in which the HSV-1-TK gene is regulated by the strong, constitutive retrovirus LTR promoter. C6-BUI cells were infected with this vector and selected for TK activity by growth in HAT medium. Parental and infected cells were tested for their dose-dependent sensitivity to gancyclovir in culture and *in vivo* following inoculation into the rat subrenal capsule.

### MATERIALS AND METHODS

Vector Construction: A 2.8 kb BamHI fragment encompassing the full coding sequence, and 2 kb of the 3' non-coding region (including the polyA addition site) of the HSV-1 TK gene (from plasmid pBRTK), was cloned into the BamHI site of a retroviral plasmid, pL(X)RNL. The resulting plasmid is called pLTKRNL. The pL(X)RNL plasmid is derived from Moloney murine leukemia retrovirus (MoMLV) and Moloney murine sarcoma retrovirus (MoMSV), and contains the following elements: a retroviral packaging sequence, psi: the neomycin-resistance (neo^{R}) gene from transposon Tn5 placed under the transcriptional control of a Rous sarcoma virus (RSV) promoter, the colE1 bacterial origin of replication: and the bacterial ampicillin resistance gene. The plasmid is basically similar to those reported in Wolff *et al., Proc. Natl. Acad. Sci.* *USA 86*:9011-9014 (1989); Short *et al., Devel. Neurosci. 12*:34-45 (1990); and Price *et al., Proc. Natl. Acad. Sci. USA 84*:156-160 (1987); except that it uses an RSV promoter to drive neo^{R}.

The BAG retroviral vector contains the *Escherichia coli lacZ* gene under the transcriptional control of a retroviral LTR promoter, the transposon Tn5 neo^{R} gene under the transcriptional control of the SV40 early promoter-enhancer element, and other features as above (Price *et al., Proc. Natl. Acad. Sci. USA 84*:156-160 (1987)).

Cell Culture: An ecotropic retrovirus-packaging line, psi2, was used which was derived from a mouse fibroblast line (Mann *et al., Cell 33*:153-159 (1983)). The C6 rat glioma-derived cell lines used were: C6-BU1 (Amano *et al., Exp. Cell Res. 85*:399-408 (1974)), a line selected in BUdR for loss of endogenous thymidine kinase activity: and C6-BUI-BAG, a derivative of C6-BUI expressing β-galactosidase activity following infection with the BAG virus. The psi2-derived line psi2-BAG-2-14 (Short *et al., Dev. Neurosci. 12*:34-45 (1990)) was used to obtain BAG virus. All cell lines were grown in Dulbecco's modified Eagle medium (GIBCO) containing 10% fetal bovine serum (FBS brand), 100 units of penicillin and 100 ug of streptomycin per ml. Neomycin-resistant cells were selected and maintained in the same medium supplemented with 1mg/ml G418 (neomycin analog, GIBCO). Cells expressing HSV-1-TK were selected by including HAT (hypoxanthine-aminopterin-thymidine, GIBCO) in the growth medium.

Transfections, Virus Production and Infections: To produce replication-defective, HSV-1-TK-bearing retroviral vectors (v-TK), 10 ug of pLTKRNL plasmid DNA were transfected into psi2 cells by the calcium phosphate co-precipitation method using glycerol shock by standard method. Transfected psi2 colonies were selected in medium containing G418. To make virus stocks, cultures were maintained in medium with G418 until they reached 80% confluency, then they were fed medium without G418 and twenty-four hours later, the virus-containing ("conditioned") medium was removed, filtered through a 0.45 µm pore size filter and stored at -70°C.

All infections were done by replacing medium on a 100 mm tissue culture dish of recipient cells with 2 ml of medium containing 4 ug/ml polybrene (Sigma) and varying amounts of virus stock.

Virus titers of the psi2-v-TK line was determined by infecting C6-BUI cells, and determining the number of HAT-resistant colonies obtained per unit volume of virus stock. Two HAT-resistant clones, C6TK-vTK1 and 3, were used for further studies. For the psi2-BAG lines, virus titers were determined the same way, using NIH3T3 cells, and selecting for G418 resistance.

Histochemical, Staining for β-galactosidase: To visualize β-galactosidase expression, cells were fixed in 0.5% gluteraldehyde in phosphate-buffered saline, pH 7.3, for 5 minutes at room temperature, and then stained with 5-bromo-4-chloro-3-indoyl-B-D-galactosidase for 30 minutes to 4 hours at 37°C (Tumer and Cepko, *Nature 328*:131-136 (1987)).

Gancyclovir Sensitivity Assays in Culture: The following cell lines; C6, CC6-BU1, C6-VIK1 and 3, were assayed for dose-dependent toxicity of the nucleoside analog gancyclovir (Cytovene, Burroughs Wellcome). Cells were plated at a density of 100 per 100 mm dish. Seventy-two hours later, gancyclovir was added at varying concentrations to each dish, and the incubation was continued for 9 days, changing the gancyclovir-containing medium every 3 days. The concentrations of gancyclovir tested were: 0, 3, 10, 30, 100, and 300µm, in triplicate. On the 9th day, the medium was removed, the dishes were washed with PBS, fixed with 100% methanol for 10 minutes, stained with a 1:10 dilution of Giemsa (Fisher) in distilled water for another 10 minutes, washed again with water, then dried (Freshney, R.I., *Culture of Animal Cells - A Manual of Basic Techniques* 2nd ed., New York, Alan R. Liss, Inc. (1987)). Colonies were counted and the number on dishes with no gancyclovir was taken to represent 100% survival.

### RESULTS

Vector Construction: The integrity and orientation of the HSV-1 TK gene in the plasmid pLTKRNL were confirmed by restriction mapping. Upon cleavage with BamHI, two bands of approximately 2.8kb and 6.7kb were obtained, as expected from the respective sizes of the HSV-1 TK gene and the pL(X)RNL vector. Based on the sequence of the HSV-1 TK gene (McKnight, S.L., *Nucleic Acids Res. 8(24)*:5949-5964 (1980)), fragments of the expected sizes were also obtained upon cleavage with the restriction endonucleases, PstI and SmaI. Insertion of the HSV-1 TX gene into the BamHI site of the pL(X)RN L vector placed it under the control of the MoMLV LTR promoter.

Transfection, Infection: The packaging line, psi2-TK produced 10^{x} cfu/ml. No helper virus production by this clone could be detected. Virus from psi2-TK was used to establish C6-derived (C6-vTK) cell lines, which grew in HAT medium.

Gancyclovir Sensitivity in Culture: The cell lines compared in the sensitivity assay were C6, C6-BU1 and C6VIK-1 and -3.

Gancyclovir Sensitivity in vivo: Nine rats were implanted in the subrenal capsule with C6VIK cells. Four survived the procedure for further study. Tumors were measured 5 days after implantation. Two animals were treated with gancyclovir (20 mg/kg intraperitoneally daily) and two with saline daily. Tumor size was reassessed over a 16-day period. The two control tumors grew four- to twelve-fold. In contrast the two treated with gancyclovir were smaller after the treatment than before.

These studies demonstrate that a retrovirus bearig the HSV-1-TK gene can be used to confer drug sensitivity on C6 glioma cell in culture and *in vivo*. This is the first retrovirus vector described bearing an active HSV-1-TK gene. It should have a number of potential uses. First, as described in detail below it should prove useful in selectively delivering this "killer" gene to tumor cells in the brain. A distinct advantage of the HSV-1-TK gene as compared to other toxic gene product is that it requires a second hit, treatment with a nucleoside analogue, to effect cell death. Further, cellular DNA replication is required for toxicity, so only dividing cells can be killed. Second, it should also be possible to use this retrovirus vector to incorporate the HSV-1-TK gene into genetically modified cells used for grafting (e.g. Rosenberg *et al., Science 242*:1575-1578 (1988). This would allow elimination of the grafted cells at a defined point in the experiment to evaluate the effect of these cells on the surrounding tissue. Third, this vector should prove useful for infecting progenitor embryonic cells to assess the nature and function of their progeny at later stages in development and throughout life. This vector, then, provides a tool to efficiently infect dividing cells in culture and *in vivo* and to insert into their genome a gene which can be used to kill them or their progeny at a defined time by application of a drug.

Primary brain tumors affect approximately 12,000 new patients in the United States each year. Twenty-five percent of primary brain tumors are glioblastomas which are only temporarily responsive or totally resistant to all forms of currently available therapy. Glioblastomas are almost universally fatal; cures remain anecdotal with only 3-5% of patients living five years beyond diagnosis. However, metastasis from glioblastoma is exceedingly rare. Glioblastomas kill from local growth, and, in many cases treated with radiation or chemotherapy, tumors recurs within 2 centimeters of the original site. This finding suggests that some tumors may be treated with a local, targetted therapeutic approach. Various attempts at local therapies have been made including photodynamic therapy (Salcman *et al., J. Neuro. Virol. 1*:225-236 (1983)), local hyperthermia (Cheng *et al., Surg. Neurol. 28*:423-435 (1986)), focal irradiation with interstitial radioisotope implants (Ortin *et al., J. Neurosurg. 67*:864-873 (1987)). To date all of these techniques have met with only limited success and have had only a marginal impact on the treatment of glioblastoma.

Because of this limited success, we decided to explore retrovirus vectors as a new avenue of potential therapy. Retroviruses take advantage of the fact that a malignant glioma is a dividing cell population with the population of non-dividing cells that compose the adult brain, thus. Retroviruses can offer a mode of selectivity for the brain tumor cells by delivering a toxic gene to them. Three toxic gene products have been used for ablation studies in transgenic mice (Bernstein and Breitman, *Mol. Biol. Med. 6*:523-530 (1989)). Two of these, ricin and diphteria toxin, however, once released into the nervous system, could cause toxicity to brain, blood vessels, bone marrow or other tissues and cells containing them could not be aborted. For this reason, we have chosen to explore a strategy of tumor cell destruction that uses the HSV-1-TK gene, that is by itself not harmful, but which sensitizes cells to exogenously administered drugs, such as gancyclovir. In this way cell destruction can be controlled.

We have demonstrated that the HSV-1-TK gene can be inserted into rat C6 glioma cells and that these cells are thereby made sensitive to gancyclovir. To demonstrate that C6 glioma cells expressing the HSV-1-TK gene can be killed *in vivo*, we used the subrenal capsule assay system in rats because it allows direct measurement of tumor volume, permits detection of small (<1mm) volume changes, and because tumor vascularization is observable and allows for entry of parenterally-administered pharmaceutical agents. This model overcomes the problems of not being able to directly observe the size of an intracerebral tumor implant and the concerns about delivery of gancyclovir through an intact blood-brain barrier. With this subrenal capsule model, we demonstrated that gancyclovir administered intraperitoneally will kill growing C6 glioma cells.

### EXAMPLE 2

Glioblastomas represent approximately 30% of primary brain tumors in adults (Schoenberg, B.S., In *Oncology of the Nervous System,* Walker, M.D. (ed.), Boston, MA: Martinus Nijhoff (1983). They are invasive, malignant, and resistant to conventional treatment modalities, and therefore are considered virtually incurable DeVita, V.T., *et al., Cancer: Principles and Practice of Oncology*, Vol. 2, 3rd ed., Philadelphia: Lippincott Press (1989); Shapiro, W.R., *et al., J. Neurosurg. 71*:1-9 (1989); Onoyama, Y., *et al., Am. J. Roentegnol. 126*:481-492 (1976); Salazar, O.M., *et al., Int. J. Rad. Oncol. Biol. Phys. 5*:1733-1740 (1979); Walker, M.D., *et al., N. Engl. J. Med. 303*:1323-1329 (1980). Recurrent disease often occurs within 2 cm of the original site (Hochberg, F.J., *et al., Neurol. 30*:907-911 (1980). With a median survival of less than a year and with only 5% of patients living after five years following diagnosis despite numerous multi-modal approaches (Mahaley, M.S., *et al., J. Neurosurg. 71*:826-836 (1989); Schoenberg, B.S., In *Oncology of the Nervous System,* Walker, M.D. (ed.), Boston, MA: Martinus Nijhoff (1983); Kim, T.S., *et al., J. Neurosurg*. (in press); Daumas-Duport, C., *et al., Cancer 62*:2152-2165 (1988)), the need for novel treatment strategies cannot be overemphasized.

One strategy is the use of viral vectors to deliver foreign genes to modulate or to destroy glioma cells. Retroviruses provide a potential means of selectively infecting tumor cells in the adult brain, because they can only integrate into the genome of dividing cells and most adult brain cells are in a quiescent, non-receptive stage of cell growth (for review of retroviruses, see Varmus, H., *Science 240*:1427-1435 (1988)). These RNA viruses have been extensively used as vectors to deliver genes to dividing cells in culture and in embryos (for review, see Cepko, C., In *Neuromethods, Vol 16, Molecular Neurobiological Techniques*, Boulton, A.A.,Boulton (eds.), Clifton, NJ: Humana (1989); Gilboa, E., *et al., BioTechniques 4*:504-512 (1986)). Foreign genes and promoter elements can be inserted into plasmid DNA equivalents of the retroviral genome, which retain the packaging signal., psi. These plasmids are then transfected into packaging cell lines, which carry wild-type retroviral sequences lacking the psi element needed for packaging of their own RNA into virion particles (Cone, R.D., *et al., Proc. Natl. Acad. Sci. USA 81*:6349-6353 (1984); Miller, A.D., *et al., Mol. Cell. Biol. 6*:2895-2902 (1986); Mann, R., *et al., Cell. 33*:153-159 (1983)). The packaging line can insert the psi-bearinng RNA encoded in the foreign gene-bearing retrovirus sequences into virion particles. These lines then release into the medium only replication-defective virions containing foreign gene sequences and no replication component virions. These replication-deficient virions can efficiently infect other dividing cells and insert the foreign genes into their genome.

A number of retroviral vectors have been developed for neuroscience applications, including ones bearing the genes for the histochemical marker, *lacZ* (Price, J., *et al., Proc. Natl. Acad. Sci. USA 84*:156-160 (1987)), nerve growth factor (Wolf, D., *et al., Mol. Biol. Med. 5*:43-59 (1988); Rosenberg, M.B., *et al., Science 242*:1575-1578 (1988)), tyrosine hydroxylase (Wolff, J.A., *et al., Proc. Natl. Acad. Sci. USA 86*:9011-9014 (1989); Horellou, P. *et al., Proc. Natl. Acad. Sci. USA 86*:7233-7237 (1989); and other proteins (Fredericksen, K., *et al., Neuron 1*:439-448 (1988); Cepko, C., In *Neuromethods, Vol. 16, Molecular Neurobiological Techniques*, Boulton, A.A.,Boulton (eds.), Clifton, NJ: Humana (1989); Cepko, C., *Ann. Rev. Neurosci. 12*:47-65 (1989)). Direct injection of *lacZ* bearing retroviruses (e.g., BAG) into embryonic tissues *in vivo* can yield gene delivery into neuroblasts and their differentiated daughter cells, as observed, for example, in epithelium, retina and cerebral cortex (Gray, G.E., *Proc. Natl. Acad. Sci. USA 85*:7356-7360 (1988); Turner, D., *et al., Nature (Lond.) 328*:131-136 (1987); Walsh, C., *et al., Science 241*:1342-1345 (1988); Luskin, M.B. *et al., Neuron 1*:635-647 (1988)). No labelling of cells has been reported following injection of this type of vector into adult nervous tissue, as anticipated from the low mitotic index of these cells and the relatively short half-life of retrovirus particles (-4 hr in culture; Cepko, C., In *Neuromethods, Vol 16, Molecular Neurobiological Techniques*, Boulton, A.A.,Boulton (eds.), Clifton, NJ: Humana (1989)). Several studies have shown that it is possible to use their retrovirus vectors for indirect "gene delivery" into adult rodent brains, by infecting dividing cells in culture and then grafting these genetically modified cells into the brain (Gage, F.H., *et al., Neuroscience 23*:795-807 (1987). This procedure has been used with the *lacZ* vector to follow the fate of grafted rat C6 glioma cells and fibroblasts (Shimohama, *S., et al., Mol. Brain Res. 5*:271-278 (1989)). Rat fibroblast lines infected with NGF and TH-bearing vectors, rat pheochromocytoma cells infected with the NGF vector, and a mouse pituitary line infected with a TII vector, have been used to deliver biologically active NGF and/or L-dopa and dopamine to regions of adult rat brain (Rosenberg, M.B., *et al., Science 242*:1575-1578 (1988); Wolff, J.A., *et al., Proc. Natl. Acad. Sci. USA 86*:9011-9014 (1989); Horellou, P., *et al., Eur. J. Neurosci. 2*:116-119 (1990)). Several new multipotent neural cell lines have been developed following infection with retrovirus vectors bearing myc and SV40T oncogenes (Snyder, E.Y., *et al., Neurosci. Abst. 9*:9 (1989); Lendahl, U., *et al., Cell 60*:585-595 (1990); Ryder, E.F., *et al., J. Neurobiol. 21*:356-375 (1990)).

In this study we have used a rodent glioma model to explore the possible use of retroviral vectors to deliver foreign genes to tumor cells *in vivo*. The BAG retrovirus vector was used to deliver the reported gene *lacZ* into rat glioma cells implanted into the adult rat brain. We have evaluated infection of endogenous brain cells and C6 glioma cells following direct injection of the BAG retrovirus or grafting of the psi 2-BAG packaging line which releases this virus vector. Cultured cells and tissue sections were evaluated by histochemical staining for bacterial beta-galactosidase, as an index of successful gene delivery, and by immunostaining for glial fibrillary acidic protein (GFAP) and S100, as a marker for glioma cells and astrocytes (Bignami, A., *et al., Brain Res. 43*:429-435 (1972)), and for fibronectin, as a marker for the fibroblast-derived packaging line.

### MATERIALS AND METHODS

Cell culture, retrovirus infection and beta-galactosidase staining. The ecotropic retrovirus producer line, ps1 2-BAG 2-14, obtained through M. Rosenberg (UCSD) from C. Cepko (Harvard Medical School) (Price, J., *et al., Proc. Natl. Acad. Sci. USA 84*:156-160 (1987); Short, M.P., *et al., Dev. Neurosci. 12*:34-45 (1990)), was grown in Dulbecco's modified Eagle's medium, 10% fetal calf serum, with 100 U/ml penicillin, 100 µg/ml streptomycin (D10 P/S), and 500 µg/ml of the neomycin analogue, G418. All cell culture materials were obtained from GIBCO. Virus was harvested by replacing the overlying media of nearly confluent cultures with a reduced volume of fresh media without G418. The conditioned media containing viral particles was removed 24-48 hr later, filtered through cellulose acetate membranes (pore size 0.45 µm, Nalgene) and stored at -70°C. The virus was tittered as colony-forming units (cfu) on 3T3 cells in the presence of G418. Viral titers were 1-3 x 10⁴ cfu/ml. In some cases, viral stock was concentrated by centrifugation (Price, J., *et al., Proc. Natl. Acad. Sci. USA 84*:156-160 (1987)) to 1-3 x 10⁵ cfu/ml.

Rat glioma cell line, C6 (Benda, P., *et al., Science 161*:370-371 (1968)), was grown in D10 P/S. A C6-BAG line was prepared by infecting C6 glioma cells with the BAG vector, and isolating single cell subclones under C418 selection. Cells were assayed for beta-galactosidase activity by histochemical analysis (Price, J., *et al., Proc. Natl Acad. Sci. USA 84*:156-160 (1987)). A subclone (C6-BAG B2-10) in which >99% of cells were beta-galactosidase positive after at least 6 passages was used in subsequent experiments at passage 2 or 3.

Cell grafting and retrovirus inoculation into adult rat brain. Adult Fischer rats weighing between 151-175 gms were anesthetized with an intraperitoneal injection of Equithesin (Short, C., *Principles and Practice of Veterinary Medicine*, Williams and Wilkins, Baltimore, MD (1987)). Two to five animals were used for each experimental paradigm, and all experiments were carried out at least twice. Stereotactic coordinates for intracerebral injection were taken from a stereotactic atlas of the adult rat (Paxinos, G., *et al.,* In *Rat Brain in Stereotaxic Coordinates*, 2nd ed., Academic Press, New York (1986)). Cells and virus were injected with 8 µg/ml polybrene, or control medium using a 10 µl Hamilton syringe with a beveled 25 gauge needle. Injections (5 µl) were done over a 5 min interval., and the needle was kept in place for another 2 min prior to removal. Surgery was tolerated well by most animals; only a few animals died during anesthesia.

For grafting experiments, confluent cultures were rinsed with Dulbecco's phosphate-buffered saline (PBS) without Ca⁺⁺ and Mg⁺⁺ and incubated with 0.05% trypsin. Cells were dispensed in D10 and pelleted by centrifugation for 5 min at 1200 x g. Cell pellets were resuspended in PBS and collected by centrifugation. Final cell suspensions were made at a density of 10⁵ cells/µl in complete PBS (PBS, which contains 1 µg/ml each MgCl₂ and CaCl₂, 0.1% glucose, and 5% rat serum (GIBCO)) and maintained at 4°C until implantation.

Tissue preparation. Prior to perfusion, rats were anesthetized with Equithesin (Short, C., *Principles and Practice of Veterinary Medicine*, Williams and Wilkins, Baltimore, MD (1987)). Perfusion was done via the ascending aorta with 50 ml of cold PBS containig 10,000 units/ml sodium heparin followed by 250 ml of cold 3% paraformaldehyde in PBS. After post fixation overnight at 4°C, brains were kept in increasing percentages (15, 20, 30) of sucrose at 4°C until they sank. Brains were frozen on dry ice and kept at -80°C until sectioning. Sections were either cut at 40 um on a freezing microtome and kept at 4°C in 0.5 M Tris-HCl, pH 7.4, with 0.1% sodium azide or in 33% polyethylene glycol until staining; or cut at 10-15 µm on a cryostat and mounted directly onto gelatin-subbed (Fisher; 100 Bloom) slides and stored at 4°C with desiccant until staining.

Histology. For beta-galactosidase histochemistry of tissues (and cells), a modification of the method of Turner and Cepko (Turner, D., *et al., Nature (London) 328*:131-136 (1987)) was used. Briefly, 5-bromo-4-chloro-3-indoyl-B-D-galactoside (X-gal., Boehringer Mannheim) was prepared as a 4% stock solution in DMSO. Free-floating or mounted sections (or cells on tissue culture dishes) were incubated at 37°C in a solution of PBS containing 2 mM MgCl₂, 35 mM K₃Fe(CN)₆, 35 mM K₄Fe(CN)₆, 0.01% sodium deoxycholate, and 0.02% NP4O, pH 7.3; 0.1% X-gal was added just prior to incubation. After incubation overnight at 37°C, cultured cells were viewed directly and sections were rinsed with PBS, mounted on subbed slides and then counterstained with hematoxylin and eosin or neutral red. They were then rinsed in water, cleared in increasing concentrations of alcohol, and placed in water prior to coverslipping with aqueous mounting media, Crystal Mount (Biomedia) or placed in xylene prior to coverslipping with Permount (Fisher).

Some sections were also stained immunocytochemically to identify GFAP, S100 or fibronectin. The sections were rinsed in PBS, incubated for 30 min with blocking serum and then overnight at room temperature with the following antibodies: mouse monoclonal antibodies to human GFAP (Boehringer Mannheim), diluted 1:3; rabbit polyclonal antibody to bovine S100 (Dako) diluted 1:750; or mouse monoclonal antibody to human fibronectin (Cappell) diluted 1:80; all of which crossreact with their respective rat antigens. Antibodies were diluted in 10 mM phosphate buffer, pH 7.4, containing 0.9% NaCl, 0.25% Triton-X and 3% blocking serum. After thorough rinsing the sections were incubated for 2 hr with either biotinylated horse antimouse IgG, biotinylated goat antirabbit IgG, or rabbit antigoat IgG (Vectastain) diluted 1:200 in the buffer, followed by several rinses in PBS. The sections were then incubated for 30 min with a complex of avidin and biotinylated horseradish peroxidase (Vectastain, ABC elite kit) diluted 1.5:100 in the buffer. The peroxidase was visualized by reacting with 0.05% 3,3-diaminobenzidine tetrahydrochloride, 0.04% NiCl₂ and 0.01% H₂O₂ in 50 mM Tris-HCl, pH 7.3, for 5-10 min at room temperature. In some cases, sections were initially stained histochemically for beta-galactosidase activity and then immunostained for GFPA. In other cases, serial selections were stained alternatively for beta-galactosidase and GFAP or S100.

### RESULTS

Histochemical staining of psi 2-BAG cells in culture demonstrated nearly 100% positive staining for beta-galactosidase and no staining for GFAP, while most C6 cells stained positively for GFAP antigen, and all were negative for beta-galactosidase staining under the neutral conditions used. The ability of the psi 2-BAG cells to release BAG virus that could infect C6 cells was demonstrated by placing coverslips containing each of these two cell types at separate locations within the same culture dish. In the presence of psi 2-BAG cells, an ever-increasing percentage of cells on the coverslip bearing C6 cells stained positively for beta-galactosidase over a 96-hr period. Essentially, all cells on the glioma coverslip were also GFAP-positive. This is consistent with successful integration of the BAG virus released by psi 2-BAG cells into glioma cell genomes.

The efficiency of gene transfer to endogenous brain cells *in vivo* was tested by direct inoculation of 5 µl BAG retrovirus vector (90-900 cfu) into adult rat hippocampus or caudate. Control animals were similarly inoculated with complete PBS. Animals were sacrificed 7 days after injections. In the animals which received direct injection of virus, as well as in control animals, no cells positive for beta-galactosidase were seen within the parenchyma. Some sections from both groups revealed faint positive staining for beta-galactosidase within the choroid plexus, as noted previously (Shimohama, S., *et al., Mol. Brain Res. 5*:271-278 (1989)). In these control sections the stain was qualitatively different than in animals in which positively staining cells are present within the tumor mass (see below).

The efficiency of direct inoculation of tumor cells in the brain was tested at varying intervals between the time of the C6 cell implants and injection of the virus, under the assumption that the glioma cells might experience a growth lag after inoculation and thus not initially be in a stage of cell division appropriate for viral integration. The site of implantation and infection was the right frontal lobe. For simultaneous injections of glioma cells and BAG virus, C6 cells (5 x 10⁵) were suspended in 5 µl of viral stock (90-900 cfu). Other animals received delayed injections of virus stock into the previous site of the C6 cell implant. Five µl aliquots of virus stock were injected using the same stereotactic coordinates with which the C6 cells had been implanted 3 and 5 days previously. Control animals received grafts of C6 or C6-BAG cells without virus. All animals were sacrificed seven to 10 days after the last viral injection. In simultaneous injections of C6 cells and BAG virus, only a few tumor cells (less than 0.1%) stained for beta-galactosidase activity. In some cases, stained endothelial cells were also noted in vessels within and around the tumor mass. In the animals in which there was a delay between the tumor implant and the virus injection, again only a few positive cells were seen. There was no notable difference between the numbers of positively staining cells in animals which had experienced a delay before the viral injection versus those which received co-injections of C6 and BAG virus. Injections of C6 and C6BAG cells gave rise to tumors of similar size. In the C6 cell injections without virus, no blue cells were seen; in the C6BAG injections, all tumor cells were positive for beta-galactosidase, as noted previously (Shimohama, S., *et al., Mol. Brain Res. 5*:271-278 (1989)).

To examine the fate of grafts of psi 2-BAG packaging cells, animals were injected with psi 2-BAG cells (5 x 10⁵ cells) into the right front lobe, and as a control, with an equal number of psi 2 cells into the left frontal lobe. Animals were sacrificed at varying times after implantation. After one day, a compact mass of beta-galactosidase positive cells were seen at the site of the right frontal injection. No positively stained cells were seen on the left side on day one, nor in either side in sections taken from animals sacrificed at days 5, 9, 14, and 21 following implantation. There was no evidence of tumor formation or other degenerative changes on the brain over this period.

The efficiency of *in situ* gene transfer of the *lacZ* gene into C6 by co-grafting of packaging line, psi 2-BAG, was then tested. For simultaneous co-grafts, the cell suspension contained a mixture of cells, in a ratio of one C6 cell to five psi 2-BAG cells. The site of implantation was again the right frontal lobe. For delayed injections, animals received implants of C6 cells (2 x 10⁵ cells) on day one, followed by injections of 5 µl of psi 2-BAG cells (5 x 10⁵ cells) on days 3 or 7. In all cases, animals were sacrificed seven days after psi 2-BAG cells had been implanted. Controls of psi 2-BAG and C6 alone were injected into other animals in parallel. In histochemically stained sections from animals which received simultaneous co-grafts of C6 and psi 2-BAG cells, both blue and non-blue cells were seen within the tumor mass. Some of these beta-galactosidase positive cells co-stained for GFAP or S100, indicating they were C6 glioma cells. There were also many GFAP or S100 positive cells within the tumor mass which were not positive for beta-galactosidase. Some of the other beta-galactosidase positive cells could be C6 cells with no or low expression of GFAP, or S100. In fact in C6 cells implanted alone into the brain, only about half of the cells in the resulting tumor mass were S100-positive and even fewer were GFAP positive. Some of the beta-galactosidase positive cells could also be psi 2-BAG cells which might have survived longer within the tumor mass as compared to the brain parenchyma; however, immunostaining for fibronectin revealed no psi 2-BAG cells in co-grafts after 7 days. Examination of serial sections of these tumors revealed many sections without any beta-galactosidase-positive cells, and our best estimate is that about 1% of the cells in the tumor expressed the *lacZ* gene in animals receiving simultaneous injection of psi 2-BAG cells and C6 cells. In contrast, sections taken from animals which had received delayed injections of the packaging line into the tumor mass contained many cells positively stained for beta-galactosidase in all sections throughout the tumor, with up to 10% of cells being positive and most positive cells at the periphery of the tumor. Co-staining for the glia-specfic antigen S100 and beta-galactosidase revealed that many of the cells within the tumor were glia-derived and some of these were also positive for beta-galactosidase activity. Tumor cells thus appear to have been more efficiently infected when the packaging line was grafted after establishment of tumor cells than when tumor and packaging cells were simultaneously injected. There did not appear to be any significant difference between animals in which the delay between injections was three days as opposed to seven days.

This study demonstrates the efficacy of a replication-defective retroviral vector in delivering the reporter gene, *lacZ*, to rat glioma cells in culture and the rat brain. In culture, the BAG retrovirus vector released from psi 2-BAG cells successfully infected C6 cells in the same dish, as demonstrated by staining for beta-galactosidase activity. The morphology and immunoreactivity to GFAP confirmed the identity of the beta-galactosidase positive cells as glioma cells. The efficiency of transfer of the *lacZ* gene to endogenous brain cells or to C6 cells *in vivo* was then compared by two techniques: direct injection of BAG virus or grafting of the packaging line which releases the virus. The highest efficiency *in vivo* was obtained by grafting of the retrovirus packaging line into an established bed of C6 tumor cells.

Initial attempts to deliver the reporter gene by direct injection of virus into the parenchyma of a normal adult rat brain produced essentially no beta-galactosidase-positive cells. In these animals, as well as in controls inoculated with complete PBS, faint positive staining was seen in the choroid plexus, but not in the parenchyma. This endogenous positive staining of lysosomal beta-galactosidase has been previously reported (Shimohama, S., *et al., Mol. Brain Res. 5*:271-278 (1989)), and was masked when sections were counterstained with neutral red. The unsuccessful direct gene delivery by the viral vector was not surprising since the majority of cells in adult rats, even in young postnatal animals, are postmitotic, and cell division is needed for retroviral integration. The site of inoculation, the hippocampus, was chosen to enhance the likelihood of successful integration, since cells in this region are the last to stop dividing after birth (Das, G.D., *et al., Brain Research 22*:122-127 (1970)). In animals inoculated with the BAG virus either simultaneously with glioma cells or after a delay following the glioma implant, only a few isolated tumor cells were successfully infected. This presumably reflects the relatively short half-life of the retrovirus *in vivo* and the state of division of the glioma cells. Of the few beta-galactosidase positive cells, most were found at the edges of the tumor where there is thought to be highest mitotic activity. Occasionally stained endothelial cells were observed, which would be expected since endothelial cells continue to divide within the blood vessels of the brain, especially in a vacularized tumor bed.

Both viral titer and the volume of the inoculum represent significant limitations to attaining a higher degree of successful integration using direct virus injection. Attempts to increase viral titer by centrifugation only increased the titer by 10- to 100-fold. When inoculating a glial tumor, which began with about 10⁵ cells, with 5 µl of a retrovirus stock of 10⁴-10⁶ cfu/ml, the ratio of virus to cell is much less than one to one (multiplicity of infection MOI) ≤ 0.01). In our hands, the efficiency of infection of rapidly dividing C6 cells in culture with the BAG retroviral vector at an MOI of 3 is approximately 30%. Thus it is not surprising that direct inoculation of the tumor was inefficient *in vivo*.

Implantation of the packaging line appears to overcome some of the limitations of direct inoculation by releasing the virus within the tumor over a prolonged period. This study demonstrates that co-grafting of the packaging line, psi 2-BAG and glioma cells, serves to deliver the reporter gene, *lacZ*, to these tumor cells more efficiently than direct viral inoculation. The efficiency was greater in animals implanted with glioma cells 3 or 7 days prior to implantation of psi 2-BAG cells as compared to simultaneous grafting of these two cell types. Histochemical analyses of sections taken from the brains of animals, which had received delayed injections, showed that large areas of the tumor were successfully infected. The brains were examined one week after the psi 2-BAG implantation, because in a separate experiment when psi 2-BAG cells were implanted alone, they were undetectable five days later. Further, immunostaining of co-grafts after 7 days revealed no fibronectin positive cells. This suggests either that the psi 2-BAG graft had been immune rejected because of a difference in rat strains or that the retroviral encoded gene, if present, was no longer being expressed (Palmer, T.D., *et al.,* personal communication). By immunocytochemistry, we have established that some of the cells within the tumor stain for both beta-galactosidase activity and GFAP or S100 antigens, confirming successful infection of glioma cells by the BAG virus released from the psi 2-BAG cells. However, there are GFAP- and S100-positive cells within the tumor which are beta-galactosidase negative, suggesting incomplete infection of tumor cells.

Several means can be envisioned to increase the efficiency of infection of glioma cells in the brain by co-grafting of retrovirus packaging lines. One way would be to carry out a series of injections of the packaging line to increase the number of cells releasing virus within the tumor bed over a longer period. Another way to increase the amount and duration of retrovirus release would be to develop a packaging line which was immune compatible with the host and thus would survive longer following grafting. Release to a larger area including the brain parenchyma surrounding the tumor might also be achieved by using an astrocyte-derived packaging line. Grafted newborn and embryonic astrocytes have been shown to be able to migrate up to 5 mm from their original site of injection and may be better than fibroblasts in reaching infiltrating glial tumor cells (Jacque *et al.* (1986); Zhou, H.F., *et al., J. Comp. Neurol. 292*:320-330 (1990); Hatton, J.D., *et al., Soc. for Neurosci. Abstracts 15*:1369 (1989)). Additionally, a glial-derived packaging line derived from glia which are endogenous brain cells, may have enhanced survival., and may be more responsive to *in situ* cues. In the case of spontaneous brain tumors, one could envision a scheme in which the tumor mass was removed, leaving some tumor cells behind, and the packaging line grafted directly into the lesion. This would serve to increase the number of packaging cells that could be grafted in and the ratio of packaging cells to tumor cells, and hence increase the ability to infect tumor cells.

This study represents a model system that could be used to deliver genes with therapeutic potential to malignant glial tumors of the central nervous system (CNS), which at this time continue to pose a unique challenge in oncology. Complete surgical extirpation is impossible, since the tumor cells infiltrate within the normal brain. Radiation therapy is limited by the sensivity of the normal brain to radiation damage. Chemotherapy is hampered by the presence of a blood brain barrrier decreasing the usefulness of agents unable to cross this barrier to reach infiltrating tumor cells. Retroviruses represent potential therapeutic agents which can confer genetic susceptibility onto tumor cells. One example would be a retrovirus packaging line that releases virions containing the herpex simplex virus thymidine kinase (HSV-TK) gene (Moolten, F.L., *et al., J. Natl. Cancer Inst. 82*:297-300 (1990)). When integrated into the mammalian cell genome, the HSV-TK gene confers sensitivity to chemotherapeutic agents, such as the nucleoside analogues, acyclovir, gancyclovir, and FIAU (Borrelli, E., *et al., Nature 339*:538-541 (1989); Moolten, *F., Cancer Res. 46*:5276-5281 (1986)). Cell culture studies have shown that C6 glioma cells and other cells infected with this retrovirus are killed at concentrations of gancyclovir 100-fold less than those required to kill uninfected cells (Moolten, F.L., *et al., J. Natl. Cancer Inst. 82*:297-300 (1990)).

It may also be possible to kill C6 glioma cells by subsequent co-grafting of the HSV-TK virus packaging line and treatment of animals with a nucleoside analogue. Further, tumor vessels may be an additional target for a proposed killing system using the HSV-TK gene.

### Example 3

Retroviral vectors can be used to transfer genes into the genome of dividing cells. In order to increase the efficiency of gene delivery and killing effect of gancyclovir, we developed a new packaging line (C6VIK-WT) by infecting C6VIK cells with an ecotropic wild-type retrovirus (MoMLV). See, Mann *et al., Cell 33*:153-159 (1983); Price *et al., Proc. Natl. Acad. Sci. USA 84*:156-160 (1987); and Cepko, C., In *Neuromethods: Molecular Neurobiological Techniques,* Vol. 16, Boulton *et al.,* eds. (Clifton, NJ, Humana), pp. 177-219. Because tumor cells can migrate deep into brain parenchyme, they should be able to deliver the vector to tumor cells away from the tumor mass. In culture, 50% of C6VIK-WT cells were killed at 0.024 µM GCV, while it took 7.3 µM GCV to kill 50% of C6VIK cells. This suggest that C6VIK-WT cells may have more HSV-TK activity than C6VIK due to multiple integrations of the HSV-TK gene or to an increased sensitivity of C6VIK-WT cells to GCV toxic products. When C6VIK and V6VIK-WT were cultured with C6BAG cells (labelled with the *lacZ* gene and thus detectable by beta-galactosidases histochemistry), following GCV treatment substantially more C6BAG cells were killed when co-cultured with C6VIK-WT than with C6VIK cells. Presumably C6VIK-WT cells produce both wild-type retrovirus and retrovirus vectors containing the HSV-TK gene (neither of which are produced by C6VIK cells) and death of C6BAG cells might be mediated by retrovirus infection and/or self-generated GCV toxic products. *In vivo* GCV treatment caused regression of tumors in most nude mice inoculated subcutaneously with C6VIK-WT cells, or with C6VIK-WT and C6BAG cells simultaneously but not with C6BAG cells alone. These findings suggest the efficiency of retrovirus-mediated gene delivery and the sensitivity to toxic agents of tumor cells can be increased using helper virus, which turns cells infected with retrovirus vectors into packaging cell lines.

The use of retroviral vectors for gene delivery need not be restricted to gene systems designed for tumor destruction. Delivery of genes involved in tumorigenesis or tumor modulation may also be a useful strategy to explore. The loss of heterozygosity for DNA markers on chromosomes 17, 10 and, less commonly, chromosome 22, in glial tumors suggests that tumor suppres or genes reside in these chromosomal regions (Bigner, S.H., *et. al., Hereditas 101*:103-113 (1984); Bigner, S.H., *et al., Cancer Res. 88*:405-411 (1988); James, CD., *et al., Cancer Res. 48*:5546-5551 (1988); El-Azouzi, M., *et al., Proc. Natl. Sci. USA 86*:7186-7190 (1989)). Restoration of retinoblastoma gene function has been shown to inhibit growth of retinoblastoma and osteosarcoma cells in culture (Huang, H.-J.S., *et al., Science 242*:1563-1566 (1988)).

Modifications of the above-described modes for carrying out the invention that will be known or can be routinely inferred by persons of skill in medicine, immunology, hybridoma technology, pharmacology, and/or related fields are intended to be within the scope of the following claims.

## Claims

1. The use of retroviral vector-producing packaging cells in the preparation of an anti-neoplastic cell agent or of a sensitizing agent against neoplastic cells already existing in a patient, wherein the retroviral vector produced by said packaging cells can infect neoplastic cells, and wherein said retroviral vector contains a gene whose gene product is capable of:
1) killing the neoplastic cells, or
2) sensitizing the neoplastic cells so that they can be killed by additional chemical treatment or radiation.

2. The use according to claim 1, wherein the neoplastic cells comprise cells of a nervous system tumour.

3. The use according to claim 2, wherein the nervous system tumour is a central nervous system tumour.

4. The use according to claim 3, wherein the central nervous system tumour is an astrocytoma, oligodendroglioma, meningioma, neurofibroma, ependymoma, Schwannoma, neurofibrosarcoma and/or especially a glioblastoma.

5. The use according to any of claims 1 to 4, wherein the gene product is thymidine kinase and chemical treatment is treatment with ganciclovir.

6. The use according to any of claims 1 to 5, wherein a helper virus is also provided to augment gene delivery.

7. The use according to any preceding claim, wherein the packaging cells are combined with a chemical suitable for use in said chemical treatment.

8. Retroviral vector-producing packaging cells for use in medicine, wherein the retroviral vector produced by said packaging cells can infect neoplastic cells already existing in a patient, and wherein said retroviral vector contains a gene whose gene product is capable of:
1) killing the neoplastic cells, or
2) sensitizing the neoplastic cells so that they can be killed by additional chemical treatment or radiation.

9. Retroviral vector-producing packaging cells for use in medicine according to claim 8, wherein said gene product is thymidine kinase and said chemical treatment is treatment with ganciclovir.

10. Retroviral vector-producing packaging cells for use in medicine according to claim 8 or claim 9 comprising or combined with a helper virus.

11. Retroviral vector-producing cells for use in medicine according to any of claims 8 to 10 combined with a chemical for use in said chemical treatment.

12. A pharmaceutical composition comprising:
a) retroviral vector-producing packaging cells, wherein the retroviral vector produced by said packaging cells can infect neoplastic cells already existing in a patient, and wherein said retroviral vector contains a gene whose gene product is capable of:
1) killing the neoplastic cells, or
2) sensitizing the neoplastic cells so that they can be killed by additional chemical treatment or radiation, and
b) a pharmaceutically acceptable (e.g. sterile) carrier.

13. A pharmaceutical composition according to claim 12 which is an injectable solution.

14. A pharmaceutical composition according to claim 12 or claim 13, wherein the gene product is thymidine kinase and the chemical treatment is treatment with ganciclovir.

15. A product comprising:
a) retroviral vector-producing packaging cells, wherein the retroviral vector produced by said packaging cells can infect neoplastic cells already existing in a patient, and wherein said retroviral vector contains a gene whose gene product is capable of:
1) killing the neoplastic cells, or
2) sensitizing the neoplastic cells so that they can be killed by additional chemical treatment or radiation, and
b) a chemical for use in said chemical treatment,
as a combined preparation for simultaneous, sequential or separate use in tumour therapy.

16. The product of claim 15, further comprising a helper virus.

17. The product of any of claims 15 or 16, wherein the gene product is thymidine kinase and the chemical is ganciclovir.

18. A process for preparing a pharmaceutically acceptable composition according to any of claims 12 to 14, comprising admixing the retroviral vector producing packaging cells and the pharmaceutically acceptable carrier.

## Patentansprüche

1. Verwendung von Retrovirusvektor-produzierenden Packungszellen in der Zubereitung einer antineoplastischen Zellsubstanz oder einer sensibilisierenden Substanz gegen neoplastische Zellen, die bereits in einem Patienten vorhanden sind, wobei der Retrovirusvektor, der von den Packungszellen erzeugt wird, neoplastische Zellen infizieren kann, und wobei der Retrovirusvektor ein Gen enthält, dessen Genprodukt imstande ist:
1) die neoplastischen Zellen abzutöten, oder
2) die neoplastischen Zellen zu sensibilisieren, so daß sie durch zusätzliche chemische Behandlung oder Strahlung abgetötet werden können.

2. Verwendung nach Anspruch 1, wobei die neoplastischen Zellen Zellen eines Nervensystemtumors umfassen.

3. Verwendung nach Anspruch 2, wobei der Nervensystemtumor ein Zentralnervensystemtumor ist.

4. Verwendung nach Anspruch 3, wobei der Zentralnervensystemtumor ein Astrocytom, Oligodendrogliom, Meningiom, Neurofibrom, Ependymom, Schwannom, Neurofibrosarcom und/oder insbesondere ein Glioblastom ist.

5. Verwendung nach einem der Ansprüche 1 bis 4, wobei das Genprodukt Thymidinkinase ist und die chemische Behandlung eine Behandlung mit Gancyclovir ist.

6. Verwendung nach einem der Ansprüche 1 bis 5, wobei auch ein Helfervirus zur Erhöhung der Genbildung bereitgestellt ist.

7. Verwendung nach einem der vorangehenden Ansprüche, wobei die Packungszellen mit einer Chemikalie kombiniert sind, die zur Verwendung in der genannten chemischen Behandlung geeignet ist.

8. Retrovirusvektor-produzierende Packungszellen zur Verwendung in der Medizin, wobei der Retrovirusvektor, der von den Packungszellen erzeugt wird, neoplastische Zellen infizieren kann, die bereits in einem Patienten vorhanden sind, und wobei der Retrovirusvektor ein Gen enthält, dessen Genprodukt imstande ist:
1) die neoplastischen Zellen abzutöten, oder
2) die neoplastischen Zellen zu sensibilisieren, so daß sie durch zusätzliche chemische Behandlung oder Strahlung abgetötet werden können.

9. Retrovirusvektor-produzierende Packungszellen zur Verwendung in der Medizin nach Anspruch 8, wobei das Genprodukt Thymidinkinase ist und die chemische Behandlung eine Behandlung mit Gancyclovir ist.

10. Retrovirusvektor-produzierende Packungszellen zur Verwendung in der Medizin nach Anspruch 8 oder 9, umfassend ein Helfervirus oder kombiniert mit diesem.

11. Retrovirusvektor-produzierende Packungszellen zur Verwendung in der Medizin nach einem der Ansprüche 8 bis 10, kombiniert mit einer Chemikalie zur Verwendung in der genannten chemischen Behandlung.

12. Pharmazeutische Zusammensetzung, umfassend:
a) Retrovirusvektor-produzierende Packungszellen, wobei der Retrovirusvektor, der von den Packungszellen erzeugt wird, neoplastische Zellen infizieren kann, die bereits in einem Patienten vorhanden sind, und wobei der Retrovirusvektor ein Gen enthält, dessen Genprodukt imstande ist:
1) die neoplastischen Zellen abzutöten, oder
2) die neoplastischen Zellen zu sensibilisieren, so daß sie durch zusätzliche chemische Behandlung oder Strahlung abgetötet werden können, und
b) einen pharmazeutisch annehmbaren (z.B. sterilen) Träger.

13. Pharmazeutische Zusammensetzung nach Anspruch 12, die eine injizierbare Lösung ist.

14. Pharmazeutische Zusammensetzung nach Anspruch 12 oder 13, wobei das Genprodukt Thymidinkinase ist und die chemische Behandlung eine Behandlung mit Gancyclovir ist.

15. Produkt, umfassend:
a) Retrovirusvektor-produzierende Packungszellen, wobei der Retrovirusvektor, der von den Packungszellen erzeugt wird, neoplastische Zellen infizieren kann, die bereits in einem Patienten vorhanden sind, und wobei der Retrovirusvektor ein Gen enthält, dessen Genprodukt imstande ist:
1) die neoplastischen Zellen abzutöten, oder
2) die neoplastischen Zellen zu sensibilisieren, so daß sie durch zusätzliche chemische Behandlung oder Strahlung abgetötet werden können, und
b) eine Chemikalie zur Verwendung in der chemischen Behandlung,
als Kombinationspräparat zur gleichzeitigen, aufeinanderfolgenden oder getrennten Verwendung in der Tumortherapie.

16. Produkt nach Anspruch 15, ferner umfassend ein Helfervirus.

17. Produkt nach Anspruch 15 oder 16, wobei das Genprodukt Thymidinkinase ist und die Chemikalie Gancyclovir ist.

18. Verfahren zur Herstellung einer pharmazeutisch annehmbaren Zusammensetzung nach einem der Ansprüche 12 bis 14, umfassend das Vermischen der Retrovirusvektor-produzierenden Packungszellen und des pharmazeutisch annehmbaren Trägers.

## Revendications

1. Utilisation de cellules de packaging productrices de vecteurs rétroviraux pour la préparation d'un agent cellulaire antinéoplastique ou d'un agent sensibilisateur contre des cellules néoplastiques existant déjà chez un patient, dans laquelle le vecteur rétroviral produit par lesdites cellules de packaging peuvent infecter des cellules néoplastiques et dans lesquelles ledit vecteur rétroviral contient un gène dont le produit génique est capable :
1) de détruire les cellules néoplastiques, ou
2) de sensibiliser les cellules néoplastiques de telle sorte qu'elles puissent être détruites par un traitement chimique ou des rayons d'appoint.

2. Utilisation selon la revendication 1, dans laquelle les cellules néoplastiques comprennent des cellules d'une tumeur du système nerveux.

3. Utilisation selon la revendication 2, dans laquelle la tumeur du système nerveux est une tumeur du système nerveux central.

4. Utilisation selon la revendication 3, dans laquelle la tumeur du système nerveux central est un astrocytome, un oligodendrogliome, un méningiome, un neurofibrome, un épendymoma, un Schwannome, un neurofibrosarcome et/ou en particulier un glioblastome.

5. Utilisation selon l'une quelconque des revendications 1 à 4, dans laquelle le produit génique est la kinase de thymidine et le traitement chimique se fait par le gancyclovir.

6. Utilisation selon l'une quelconque des revendications 1 à 5, dans laquelle un virus auxiliaire est également prévu pour augmenter la délivrance du gène.

7. Utilisation selon l'une quelconque des revendications précédentes, dans laquelle les cellules de packaging sont combinées avec un produit chimique convenant à une utilisation dans ledit traitement chimique.

8. Cellules de packaging productrices de vecteurs rétroviraux pour un usage en médecine, dans lesquelles le vecteur rétroviral produit par lesdites cellules de packaging peut infecter des cellules néoplastiques déjà existant chez un patient, et dans lesquelles ledit vecteur rétroviral contient un gène dont le produit génique est capable :
1) de détruire les cellules néoplastiques ou
2) de sensibiliser les cellules néoplastiques de telle sorte qu'elles puissent être détruites par un traitement chimique ou des rayons d'appoint.

9. Cellules de packaging productrices de vecteurs rétroviraux pour un usage en médecine selon la revendication 8, dans lesquelles ledit produit génique est la kinase de thymidine et ledit traitement chimique est un traitement par du gancyclovir.

10. Cellules de packaging productrices de vecteurs rétroviraux susceptibles d'être utilisées en médecine selon la revendication 8 ou 9 comprenant un virus auxiliaire ou combinées à un virus auxiliaire.

11. Cellules productrices de vecteurs rétroviraux pour un usage en médecine selon l'une quelconque des revendications 8 à 10 combinées à un produit chimique pour un usage dans ledit traitement chimique.

12. Composition pharmaceutique comprenant :
a) des cellules de packaging productrices de vecteurs rétroviraux, dans lesquelles le vecteur rétroviral produit par lesdites cellules de packaging peut infecter des cellules néoplastiques existant déjà chez un patient et dans lesquelles ledit vecteur rétroviral contient un gène dont le produit génique est capable :
1) de détruire les cellules néoplastiques ou
2) de sensibiliser les cellules néoplastiques de telle sorte qu'elles puissent être détruites par un traitement chimique ou des rayons d'appoint, et
b) un véhicule acceptable au plan pharmaceutique (par exemple stérile).

13. Composition pharmaceutique selon la revendication 12 qui est une solution injectable.

14. Composition pharmaceutique selon la revendication 12 ou 13, dans laquelle le produit génique est la kinase de thymidine et le traitement chimique est le traitement par le gancyclovir.

15. Produit comprenant :
a) des cellules de packaging productrices de vecteurs rétroviraux, dans lesquelles le vecteur rétroviral produit par lesdites cellules de packaging peut infecter des cellules néoplastiques existant déjà chez un patient et dans lesquelles ledit vecteur rétroviral contient un gène dont le produit génique est capable :
1) de détruire les cellules néoplastiques, ou
2) de sensibiliser les cellules néoplastiques de telle sorte qu'elles puissent être détruites par un traitement chimique ou des rayons d'appoint, et
b) un produit chimique pour un usage dans ledit traitement chimique,
sous la forme d'une préparation combinée pour un usage simultané, successif ou séparé en thérapie tumorale.

16. Produit selon la revendication 15, comprenant par ailleurs un virus auxiliaire.

17. Produit selon l'une quelconque des revendications 15 ou 16, dans lequel le produit génique est la kinase de thymidine et le produit chimique est le gancyclovir.

18. Procédé de préparation d'une composition acceptable au plan pharmaceutique selon l'une quelconque des revendications 12 à 14, comprenant le mélange des cellules de packaging productrices de vecteurs rétroviraux et du véhicule acceptable au plan pharmaceutique.
